(19) Europäisches Patentamt
European Patent Office
Office européen des brevets

(11) **EP 4 582 083 A1**

(12) **EUROPEAN PATENT APPLICATION**
published in accordance with Art. 153(4) EPC

(43) Date of publication:
**09.07.2025 Bulletin 2025/28**

(21) Application number: **23860961.4**

(22) Date of filing: **04.09.2023**

(51) International Patent Classification (IPC):
*A61K 9/19* (2006.01)        *A61K 47/26* (2006.01)
*A61K 47/20* (2006.01)       *A61K 9/08* (2006.01)
*A61K 38/48* (2006.01)       *A61K 8/66* (2006.01)
*A61Q 19/00* (2006.01)

(52) Cooperative Patent Classification (CPC):
**A61K 8/66; A61K 9/08; A61K 9/19; A61K 38/48;
A61K 47/20; A61K 47/26; A61Q 19/00**

(86) International application number:
**PCT/KR2023/013194**

(87) International publication number:
**WO 2024/049286 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.09.2022 KR 20220111408**

(71) Applicant: **Medytox Inc.
Chungcheongbuk-do 28126 (KR)**

(72) Inventors:
• **RHEE, Chang Hoon
Gunpo-si Gyeonggi-do 15823 (KR)**
• **KIM, Keun Soo
Daejeon 35201 (KR)**

(74) Representative: **Brevalex
Tour Trinity
1 B Place de la Défense
92400 Courbevoie (FR)**

(54) **BOTULINUM TOXIN FORMULATION HAVING REDUCED RESISTANCE EXPRESSION, AND METHOD RELATED THERETO**

(57)    Provided are a botulinum toxin formulation including a botulinum toxin in an amount corresponding to a desired potency, a method of manufacturing the same, a cosmetic or therapeutic method, and a method of reducing immunogenicity of a botulinum toxin formulation, using the same. Therefore, the formulation according to the present disclosure has low immunogenicity and may exhibit low resistance expression when administered to a subject.

EP 4 582 083 A1

## EP 4 582 083 A1

**Description**

**Technical Field**

The present disclosure relates to a botulinum toxin formulation and a method related thereto, and more particularly, to a botulinum toxin formulation including an amount of botulinum toxin corresponding to a desired potency, a method of manufacturing the same, a cosmetic or therapeutic method using the same, and a method of reducing immunogenicity of a botulinum toxin formulation, using the same.

**Background Art**

In general, it is undesirable to use an overage of a drug substance to compensate for degradation during the manufacturing process or during the storage period of the product. Regardless of whether an overage is included in the final formulated product, the validity of an overage in the manufacturing of the drug product must be justified in consideration of the safety and efficacy of the product. Information on the quantity of the overage, the reasons for the overage (e.g., to compensate for anticipated and documented manufacturing losses), and the justification for the amount of the overage should be provided. The overage must be included in the quantity of the drug substance listed in the batch formula.

If an overage occurs during the manufacturing process, an amount greater than the target quantity of the protein will be included during the manufacturing of the final product. This means that more protein is included than is required for the final product. In this case, not only is the active protein included in the final product administered to the patient but also inactive protein is administered. Typically, exogenous proteins act as immunogens when administered into the body, triggering an immune response and the generation of neutralizing antibodies, which lead to the removal of the protein from the body. In this regard, excess protein introduced due to the overage acts as an immunogen in the body, resulting in the generation of neutralizing antibodies and, in some cases, failure to exhibit the intended efficacy. Particularly, since botulinum toxin usually requires periodic and repeated administration, the subjects receiving such treatments are continuously exposed to botulinum toxin. Therefore, to minimize the immunogenic effects of botulinum toxin formulations, it is important to formulate the final product by minimizing the overage during manufacturing.

Botulinum toxin (botulinum neurotoxin, BoNT) is a polypeptide product of the anaerobic bacterium *Clostridium botulinum.* It functions by paralyzing muscles by preventing the release of neurotransmitters at the junction between nerves and muscles. Although botulinum toxin is inherently a toxic substance that causes fatal poisoning, its therapeutic potential was first realized in treating diseases caused by excessive muscle contractions, such as strabismus. Since then, its applications have broadened significantly and are now used for various purposes. Botulinum toxin is primarily used to treat conditions such as dystonia, hemifacial spasm, tremor, spasticity, and other movement disorders unrelated to dystonia. Additionally, it is utilized for treating ocular diseases, pain-related conditions such as migraines, urologic diseases, and for cosmetic purposes such as wrinkle treatment.

The manufacturing (formulation) process for botulinum toxin pharmaceutical compositions typically requires an overage of 50 % or more. The fact that 50 % of the botulinum toxin is used as an overage in the manufacturing process of the pharmaceutical composition means that a manufacturing process starting with 1.5 times the amount (i.e., 150 units) of botulinum toxin results in a pharmaceutical composition including 1 time the amount (e.g., 100 units) of botulinum toxin. The manufacturing or formulation process involves diluting, formulating, and processing botulinum toxin obtained from bacterial fermentation (referred to as bulk or raw toxin) into a botulinum toxin pharmaceutical composition suitable for therapeutic and/or cosmetic applications in humans. Thus, it has been known that during the formulation process, 50 % or more of the botulinum toxin potency is lost for reasons that cannot be fully explained, and overage exceeding 50 % is required due to denaturation and/or loss of botulinum toxin during the formulation process.

International publication WO2015-089452 relates to a botulinum toxin composition free of animal proteins and their use in patients requiring treatment for diseases, disorders, or abnormalities. This publication discloses that botulinum toxin compositions free of animal proteins extend the efficacy of botulinum toxin as compared to compositions including animal proteins. However, it does not disclose or suggest anything about the overage of botulinum toxin.

International publication WO2007-016018 provides pharmaceutical compositions that eliminate the need for botulinum toxin overage by optimizing the ratio of excipients and performing a vacuum-drying process under appropriate conditions. Specifically, the composition includes botulinum toxin, a first excipient (albumin), and a second excipient (e.g., sodium chloride), with the ratio of the first excipient to the second excipient in the pharmaceutical composition more than 0.6 and less than approximately 100. Notably, it discloses a pharmaceutical composition where the weight ratio of human serum albumin (HSA) to NaCl in Botox® is increased from 0.6 to 28, which is a 47-fold increase. However, this composition includes albumin, an animal protein.

**Disclosure of Invention**

# EP 4 582 083 A1

**Technical Problem**

An object of the present disclosure is to provide a botulinum toxin formulation including an amount of botulinum toxin corresponding to a desired potency, while being free of albumin.

An object of the present disclosure is to provide a method of manufacturing the botulinum toxin formulation.

An object of the present disclosure is to provide a method of reducing a potency loss of botulinum toxin during the manufacturing process using the botulinum toxin formulation.

An object of the present disclosure is to provide a method of treating various diseases or improving wrinkles by administering the botulinum toxin formulation to a subject.

An object of the present disclosure is to provide a method of reducing the immunogenicity of a botulinum toxin formulation by administering the botulinum toxin formulation to a subject.

**Solution to Problem**

An aspect of the present disclosure relates to a botulinum toxin formulation free of animal protein, including an amount of botulinum toxin corresponding to a desired potency,

wherein the amount corresponding to the desired potency includes an overage of less than 50 %.

In an embodiment, the formulation may include a smaller amount of the overage of botulinum toxin compared to a botulinum toxin formulation including animal proteins.

In an embodiment, the botulinum toxin formulation may be a lyophilized formulation or a liquid formulation.

In an embodiment, the lyophilized formulation may include a botulinum toxin, a surfactant, an amino acid, and one or more components selected from the group consisting of sugars, sugar alcohols, and ionic compounds. For example, the amino acid may include methionine. For example, the surfactant may include one or more of a polysorbate and a poloxamer.

In an embodiment, the liquid formulation may include a botulinum toxin, a surfactant, and an amino acid. For example, the amino acid may include methionine. For example, the surfactant may include one or more of a polysorbate and a poloxamer.

In an embodiment, the liquid formulation may include a surfactant in an amount of 0.05 mg/ml to 50 mg/ml and an amino acid in an amount of 0.05 mg/ml to 8 mg/ml.

In an embodiment, the liquid formulation may include a botulinum toxin, a surfactant, and an amino acid.

In an embodiment, the surfactant may include one or more of a polysorbate and a poloxamer.

In an embodiment, the botulinum toxin may be selected from the group consisting of botulinum toxin serotypes A, B, C, D, E, F, and G. For example, the botulinum toxin may be botulinum toxin serotype A.

In an embodiment, the botulinum toxin formulation may be intended for improving symptoms such as wrinkles, a square jaw, a pointed jaw, wounds, skin softening, scars, acne, pores, elasticity, or keloids. Additionally, it may be used for treating facial spasms, eyelid spasms, torticollis, blepharospasm, cervical dystonia, oropharynx dystonia, spasmodic dysphonia, migraines, pruritis ani, or hyperhidrosis.

A second aspect of the present disclosure relates to a method of manufacturing a botulinum toxin formulation, including mixing a botulinum toxin with a pharmaceutically acceptable excipient to obtain a botulinum toxin formulation,

wherein the pharmaceutically acceptable excipient does not include albumin,
wherein the botulinum toxin is used in an amount corresponding to a desired potency, and
wherein the amount corresponding to the desired potency includes an overage of less than 50 %.

In an embodiment, the mixing a excipient may include mixing 0.05 mg/ml to 50 mg/ml of a surfactant and mixing 0.05 mg/ml to 8 mg/ml of an amino acid.

A third aspect of the present disclosure relates to a method of reducing a potency loss of a botulinum toxin during a manufacturing process, which includes obtaining the botulinum toxin formulation.

A fourth aspect of the present disclosure relates to a method of reducing the immunogenicity of a botulinum toxin formulation, which includes administering an effective amount of the botulinum toxin formulation according to the present disclosure to a subject in need thereof.

A fifth aspect of the present disclosure relates to a method of improving symptoms such as wrinkles, a square jaw, a pointed jaw, wounds, skin softening, scars, acne, pores, elasticity, or keloids; or for treating facial spasms, eyelid spasms, torticollis, blepharospasm, cervical dystonia, oropharynx dystonia, spasmodic dysphonia, migraines, pruritis ani, or hyperhidrosis. The method includes administering an effective amount of the botulinum toxin formulation according to the present disclosure to a subject in need thereof.

A sixth aspect of the present disclosure relates to a use of an effective amount of a botulinum toxin formulation according to the present disclosure in the manufacture of a medicament for improving symptoms such as wrinkles, a square jaw, a pointed jaw, wounds, skin softening, scars, acne, pores, elasticity, or keloids; or for treating facial spasms, eyelid spasms, torticollis, blepharospasm, cervical dystonia, oropharynx dystonia, spasmodic dysphonia, migraines, pruritis ani, or hyperhidrosis.

**Advantageous Effects of Invention**

According to the present disclosure, a botulinum toxin formulation including an amount of botulinum toxin corresponding to a desired potency, a method of manufacturing the same, a method of reducing potency loss of botulinum toxin during the manufacturing process using the formulation, cosmetic or therapeutic methods using the formulation, and a method of reducing the immunogenicity of botulinum toxin formulations using the formulation are provided.

**Best Mode for Carrying out the Invention**

Unless defined otherwise, technical and scientific terms used herein have the same meaning as commonly used in the field to which this disclosure belongs. For the purpose of understanding the present disclosure, the following definitions shall apply, and terms used in the singular include their plural forms, and vice versa.

Terms such as "first" and "second" may be used to describe various components, but these components should not be limited by these terms. The terms are used solely to distinguish one component from another. For example, a first component may be referred to as a second component without departing from the scope of the present disclosure, and similarly, a second component may be referred to as a first component.

The term "and/or" includes all combinations that the associated constructs may define.

As used herein, the term "botulinum toxin" refers to a botulinum toxin protein molecule produced by bacteria or recombinant methods, including all serotypes and their variants or fusion proteins.

In an embodiment, botulinum toxin may be used without limitation as to serotype and may be selected from the group consisting of type A (BoNT/A), B (BoNT/B), C (BoNT/C), D (BoNT/D), E (BoNT/E), F (BoNT/F), G (BoNT/G), H (BoNT/H), X (BoNT/X), Enterococcus species botulinum toxin J (eBoNT/J), mosaic botulinum toxins, and/or their variants. Examples of mosaic toxins include BoNT/DC, BoNT/CD, and BoNT/FA. For example, type A may be used.

In an embodiment, botulinum toxin is derived from various BoNT/A subtypes, such as A1, A2, A3, A4, A5, A6, A7, A9, and A10; BoNT/B subtypes, such as B1, B2, B3, B4, B5, B6, B7, B8, Bnp, and Bbv; BoNT/C subtypes, such as C and CD; BoNT/D subtypes, such as D and DC; BoNT/E subtypes, such as E1, E2, E3, E4, E5, E6, E7, E8, and E9; BoNT/F subtypes, such as F1, F2, F3, F4, F5, F6, and F7; and BoNT/G subtypes, such as subtype G. BoNT subtypes also includes chimeric BoNTs, such as BoNT/DC, BoNT/CD, BoNT/FA, and others.

Botulinum toxin includes a botulinum toxin derivative. The botulinum toxin derivative may be a derivative of natural botulinum toxin with botulinum toxin activity or a derivative of recombinant native botulinum toxin that includes one or more chemical or functional modifications on a portion of the molecule or on one or more chains. For example, the botulinum toxin derivative may be a modified toxin having one or more amino acids deleted, altered or substituted. The botulinum toxin may be a recombinant peptide, a fusion protein, or a hybrid neurotoxin prepared, for example, from subunits or domains of different toxin serotypes. Additionally, the botulinum toxin may be a part of a whole molecule with toxin activity, a combination of such molecules, or conjugated molecules, such as components of a fusion protein.

The term "complex protein" refers to hemagglutinin and non-toxic non-hemagglutinin (NTNH) components that lack hemagglutinin activity. *Clostridium botulinum* strains produce immunologically distinct neurotoxins (types A-G). The molecular masses of type A-G neurotoxins (NTX) are approximately 150 kDa (7S). In culture media and foods with acidic conditions, NTX combines with non-toxic components to form larger complexes known as progenitor toxins (PTX). PTX may have molecular weights of 900 kDa (19S), 500 kDa (16S), and 300 kDa (12S). The 12S toxin consists of NTX and a non-toxic component (NTNH) that has no hemagglutinin activity, while the 19S and 16S toxins consist of NTX, NTNH, and hemagglutinin. Type A strains produce three forms of toxins (19S, 16S, and 12S), while types B, C, and D strains produce 16S and 12S toxins. Under alkaline conditions, PTX dissociates into NTX and non-toxic components. The 19S and 16S toxins dissociate into NTX and non-toxic components of NTNH and hemagglutinin, while the 12S toxin dissociates into NTX and NTNH.

The botulinum toxin composition of the present disclosure may be a complex botulinum toxin composition including a complex protein. For example, a complex botulinum toxin may include NTNH or hemagglutinin, in which case the molecular weight of the complex botulinum toxin may be about 900 kDa or 500 kDa.

The botulinum toxin composition of the present disclosure may be a non-complexed botulinum toxin composition that does not contain a complex protein. For example, the non-complexed botulinum toxin may lack NTNH or hemagglutinin, in which case the molecular weight of the non-complexed botulinum toxin may be about 150 kDa.

The term "animal protein" refers to products derived from blood, contaminated with blood, or originating from other animal sources.

The botulinum toxin composition of the present disclosure may be a botulinum toxin composition that does not include animal proteins. For example, the botulinum toxin composition of the present disclosure may lack a protein stabilizer derived from animals. In an embodiment, the botulinum toxin composition of the present disclosure may not include albumin, such as human serum albumin or recombinant human albumin.

The term "overage" refers to an excess amount added to compensate for the loss of the active ingredient in the drug product, and it refers to an excess amount added to compensate for losses of the active ingredient during the manufacturing process or storage period of the product. Therefore, "overage" refers to an amount of toxin added in excess, calculated based on the target toxin potency by determining the initial toxin input amount. After the manufacturing process is completed, the final potency is confirmed using a potency assay. If the final potency is lower than the target potency due to toxin protein inactivation during the process, the amount of toxin added in excess compensates for this discrepancy, considering the inactivation of the toxin protein during the manufacturing process. Therefore, overage may be calculated as follows:

$$\text{Overage (\%)} = (((100 \,/\, \text{Potency recovery rate})-1) \times 100)$$

As used herein, potency may be measured by a mouse potency assay or by a potency assay using a method that produces substantially equivalent results thereto (e.g., a cell-based potency assay).

As used herein, the terms "unit", "unit(s)", or "U" refer to $LD_{50}$ dose, defined as the amount of botulinum toxin that results in the death of 50 % of mice injected with botulinum toxin, and are used interchangeably within a product.

As used herein, "an amount corresponding to a desired potency" may refer to an amount that includes a significantly reduced overage compared to what is typically required in the manufacturing process of botulinum toxin formulations, for example, an overage of less than 50 %.

As used herein, "does not include an overage" refers to minimizing the overage required in the manufacturing process of botulinum toxin formulations, for example, less than 50 %, 45 % or less, 40 % or less, 35 % or less, 30 % or less, 25 % or less, 20 % or less, 10 % or less, 5 % or less, 3 % or less, 1 % or less, or 0.1 % or less.

As used herein, the difference between the botulinum toxin overage (%) of a botulinum toxin formulation including animal proteins and the botulinum toxin overage (%) of the formulation in an embodiment is calculated by subtracting the botulinum toxin overage (%) value of the formulation in an embodiment from the botulinum toxin overage (%) value of the botulinum toxin formulation including animal proteins. For example, the difference may be 10 or greater, 15 or greater, 20 or greater, 25 or greater, 30 or greater, 35 or greater, 40 or greater, 45 or greater, 50 or greater, 55 or greater, 60 or greater, 65 or greater, 70 or greater, 75 or greater, 80 or greater, 85 or greater, or 90 or greater.

"Pharmaceutical composition" refers to a formulation including an active ingredient. The term "formulation" refers to that, in addition to the active ingredient (e.g., botulinum toxin), at least one additional component, such as albumin (e.g., human serum albumin or recombinant human albumin) and/or sodium chloride, is present in the pharmaceutical composition. A pharmaceutical composition is a formulation suitable for administration to a subject, such as a human patient. The pharmaceutical composition may be in a lyophilized form, for example, a solution formed after reconstitution of the lyophilized pharmaceutical composition using saline or water, or it may be in a solution form that does not require reconstitution. The pharmaceutical composition may be liquid or solid. The pharmaceutical composition may be free of animal proteins and/or albumin.

The term "active ingredient" refers to a substance in a formulation or composition that is biologically or physiologically active. In particular, in the context of pharmaceutical formulations or compositions, the term "active ingredient" refers to the substance that exhibits a desired pharmacological effect. For example, the active ingredient in the present disclosure may be botulinum toxin free of complex proteins.

"Administration" or "administering" refers to providing a pharmaceutical composition or active ingredient to a subject. The pharmaceutical composition may be administered via various appropriate routes.

In an embodiment, the administration of the pharmaceutical composition may be performed via transdermal, subcutaneous, or intramuscular injection. In an embodiment, the composition may be administered locally to a muscle or group of muscles. For example, the reduction of forehead wrinkles or skin wrinkles may be achieved by administering the composition transdermally or subcutaneously into the area of the wrinkles.

"Treat", "treating" or "treatment" refer to the alleviation or reduction (including partial reduction, substantial reduction, substantially complete reduction and complete reduction), resolution or prevention (whether temporary or permanent) of a disease, disorder or condition so as to achieve a desired therapeutic result, for example, by healing injured or damaged tissue, or by altering, changing, strengthening, improving, ameliorating and/or beautifying a pre-existing or recognized disease, disorder or condition. As used herein, "treatment" is a concept that includes prevention. "Prevention" refers to the delay in the onset of a disease, disorder, or condition. If the onset of the disease, disorder, or condition is delayed for a predetermined period, prevention may be considered complete.

In an embodiment, "treatment" refers to the treatment of a disease, disorder, or medical condition in a patient, such as a mammal (particularly a human), which includes one or more of the following:

(a) preventing the occurrence of the disease, disorder, or medical condition, such as preventing the recurrence of the disease or medical condition, or prophylactic treatment of a patient pre-disposed to the disease or medical condition;

(b) ameliorating the disease, disorder, or medical condition, including antagonizing the effects of other therapeutic agents, such as eliminating or regressing the disease, disorder, or medical condition in the patient;

(c) suppressing the disease, disorder, or medical condition, such as slowing or halting the progression of the disease, disorder, or medical condition in the patient; or

(d) alleviating symptoms of the disease, disorder, or medical condition in the patient.

In an embodiment, a therapeutically effective amount of the botulinum toxin is about 0.01 U/kg to about 100 U/kg, about 0.1 U/kg to about 100 U/kg, about 0.2 U/kg to about 100 U/kg, about 0.2 U/kg to about 50 U/kg, about 0.2 U/kg to about 30 U/kg, about 0.2 U/kg to about 10 U/kg, or about 0.2 U/kg to about 1 U/kg. In another embodiment, based on a 60 kg adult body weight, the dose may be about 1 U to about 10,000 U, about 1 U to about 5,000 U, about 1 U to about 2,500 U, about 1 U to about 1,000 U, about 1 U to about 500 U, about 1 U to about 300 U, about 1 U to about 200 U, about 10 U to about 200 U, about 10 U to about 100 U, or about 10 U to about 50 U.

According to an aspect of the present disclosure,

a botulinum toxin formulation free of albumin is provided,
which includes an amount of botulinum toxin corresponding to a desired potency,
wherein the amount corresponding to the desired potency includes an overage of less than 50 %.

In an embodiment, the overage of the botulinum toxin formulation may be less than 50%, 45% or less, 40% or less, 35% or less, 30% or less, 25% or less, 20% or less, 15% or less, 10% or less, 5% or less, 3% or less, 1% or less, or 0.1% or less.

In an embodiment, the botulinum toxin formulation does not include any overage.

In an embodiment, the difference in the botulinum toxin overage (%) between a botulinum toxin formulation including animal proteins and the formulation according to an embodiment may be 40 or greater, 45 or greater, 50 or greater, 55 or greater, 60 or greater, 65 or greater, 70 or greater, 75 or greater, 80 or greater, 85 or greater, or 90 or greater.

In an embodiment, the amount of the botulinum toxin included in the botulinum toxin formulation may be 50 % to 90 %, 50 % to 85 %, 50 % to 80 %, 50 % to 75 %, 50 % to 70 %, 50 % to 65 %, 50 % to 60 %, 50 % to 55 %, 60 % to 90 %, 60 % to 85 %, 60 % to 80 %, 60 % to 75 %, 60 % to 70 %, or 60 % to 65 % of the amount of botulinum toxin included in a botulinum toxin formulation including animal-derived proteins.

In other words, the formulation according to an embodiment may include a smaller amount of botulinum toxin than the amount of botulinum toxin included in a botulinum toxin formulation including animal-derived proteins.

The formulation according to an embodiment includes a reduced absolute amount of toxin (both active and inactive toxin) compared to conventional formulations, as the overage of toxin is reduced. Therefore, when the formulation according to an embodiment is administered to a subject, the overage of immunogenic proteins may be reduced by 50 % or more, leading to a decrease in immunogenicity. This reduction suppresses the immune response in the subject's body, thereby inhibiting or reducing the generation of neutralizing antibodies. Consequently, resistance expression to botulinum toxin is decreased in subjects receiving the formulation according to an embodiment.

In an embodiment, the botulinum toxin formulation may include a pharmaceutically acceptable excipient or additive, wherein the pharmaceutically acceptable excipient or additive may include a stabilizer (e.g., amino acid), an ionic compound, a surfactant, a buffer, a lyoprotectant, or a combination thereof.

The surfactant may be a nonionic surfactant, for example, an alkylene oxide-based surfactant such as an ethylene oxide-based surfactant or a propylene oxide-based surfactant. Examples include a polyoxyethylene sorbitan fatty acid ester and/or a polyoxyethylene-polyoxypropylene block copolymer, such as a poloxamer and/or a polysorbate.

Examples include an amino acid (e.g., methionine, arginine, alanine, glutamic acid, aspartic acid, glycine, and/or cysteine, such as methionine), a salt (e.g., NaCl), a buffer, a nonionic surfactant (e.g., poloxamers and/or polysorbates, such as polysorbate 20 and/or polysorbate 80), a sugar (e.g., disaccharides such as sucrose), a sugar alcohol (e.g., sorbitol), or a combination thereof.

For example, the pharmaceutically acceptable excipient or additive may include surfactants (e.g., nonionic surfactants) and amino acids.

In an embodiment, the lyophilized formulation may include a botulinum toxin, a surfactant, an amino acid, and one or more components selected from the group consisting of sugars, sugar alcohols, and ionic compounds. For example, the surfactant may include polysorbates and/or poloxamers, and the amino acid may include methionine.

In an embodiment, the lyophilized formulation may include pharmaceutically acceptable excipients or additives in a predetermined amount.

As used herein, the content of excipients or additives included in the lyophilized formulation is described based on the content in the liquid phase before the lyophilization process.

In an embodiment, the lyophilized formulation may include a surfactant (a nonionic surfactant, e.g., an alkylene oxide-based surfactant such as an ethylene oxide-based surfactant or a propylene oxide-based surfactant, e.g., a polyoxyethylene sorbitan fatty acid ester and/or a polyoxyethylene-polyoxypropylene block copolymer, such as a poloxamer and/or a polysorbate) in an amount of from 0.01 mg/ml to 100 mg/ml, 0.01 mg/ml to 80 mg/ml, 0.01 mg/ml to 50 mg/ml, 0.03 mg/ml to 100 mg/ml, 0.03 mg/ml to 80 mg/ml, 0.03 mg/ml to 80 mg/ml, 0.05 mg/ml to 100 mg/ml, 0.05 mg/ml to 80 mg/ml, or 0.05 mg/ml to 50 mg/ml. In an embodiment, the lyophilized formulation may include an amino acid (e.g., methionine, arginine, alanine, glutamic acid, aspartic acid, glycine, and/or cysteine, such as methionine) in an amount ranging from 0.01 mg/ml to 30 mg/ml, 0.01 mg/ml to 20 mg/ml, 0.01 mg/ml to 15 mg/ml, 0.01 mg/ml to 10 mg/ml, 0.03 mg/ml to 30 mg/ml, 0.03 mg/ml to 20 mg/ml, 0.03 mg/ml to 15 mg/ml, 0.03 mg/ml to 10 mg/ml, 0.05 mg/ml to 30 mg/ml, 0.05 mg/ml to 20 mg/ml, 0.05 mg/ml to 15 mg/ml, 0.05 mg/ml to 10 mg/ml, 0.05 mg/ml to 8 mg/ml, or 0.05 mg/ml to 3 mg/ml. In an embodiment, the lyophilized formulation may include a surfactant in an amount of 0.05 mg/ml to 50 mg/ml and an amino acid in an amount of 0.05 mg/ml to 8 mg/ml. In an embodiment, the lyophilized formulation may include polysorbates or poloxamers in an amount of 0.05 mg/ml to 50 mg/ml, and amino acids in an amount of 0.05 mg/ml to 8 mg/ml.

In an embodiment, the lyophilized formulation may include sodium chloride in an amount ranging from 0.1 mg/ml to 50 mg/ml, 0.5 mg/ml to 50 mg/ml, 1 mg/ml to 50 mg/ml, 1 mg/ml to 40 mg/ml, 0.1 mg/ml to 30 mg/ml, 0.1 mg/ml to 20 mg/ml, 0.1 mg/ml to 15 mg/ml, 0.1 mg/ml to 10 mg/ml, 0.5 mg/ml to 30 mg/ml, 0.5 mg/ml to 20 mg/ml, 3 mg/ml to 15 mg/ml, 3 mg/ml to 20 mg/ml, or 5 mg/ml to 15 mg/ml.

In an embodiment, the lyophilized formulation may include sucrose in an amount ranging from 1 mg/ml to 100 mg/ml, 1 mg/ml to 90 mg/ml, 1 mg/ml to 80 mg/ml, 1 mg/ml to 70 mg/ml, 1 mg/ml to 60 mg/ml, 1 mg/ml to 50 mg/ml, 3 mg/ml to 50 mg/ml, 5 mg/ml to 50 mg/ml, 7 mg/ml to 50 mg/ml, 10 mg/ml to 50 mg/ml, 10 mg/ml to 40 mg/ml, or 20 mg/ml to 40 mg/ml.

In an embodiment, the liquid formulation may include botulinum toxin, polysorbate, and an amino acid. For example, the surfactant may include polysorbates and/or poloxamers, and the amino acid may include methionine.

In an embodiment, the liquid formulation may include a surfactant (e.g., a nonionic surfactant, such as an alkylene oxide-based surfactant like ethylene oxide-based or propylene oxide-based surfactants, for example, polyoxyethylene sorbitan fatty acid esters and/or polyoxyethylene-polyoxypropylene block copolymers, such as poloxamers and/or polysorbates) in an amount ranging from 0.01 mg/ml to 100 mg/ml, 0.01 mg/ml to 80 mg/ml, 0.01 mg/ml to 50 mg/ml, 0.03 mg/ml to 100 mg/ml, 0.03 mg/ml to 80 mg/ml, 0.03 mg/ml to 80 mg/ml, 0.05 mg/ml to 100 mg/ml, 0.05 mg/ml to 80 mg/ml, or 0.05 mg/ml to 50 mg/ml.

In an embodiment, the liquid formulation may include an amino acid (e.g., methionine, arginine, alanine, glutamic acid, aspartic acid, glycine, and/or cysteine, such as methionine) in an amount ranging from 0.01 mg/ml to 30 mg/ml, 0.01 mg/ml to 20 mg/ml, 0.01 mg/ml to 15 mg/ml, 0.01 mg/ml to 10 mg/ml, 0.03 mg/ml to 30 mg/ml, 0.03 mg/ml to 20 mg/ml, 0.03 mg/ml to 15 mg/ml, 0.03 mg/ml to 10 mg/ml, 0.05 mg/ml to 30 mg/ml, 0.05 mg/ml to 20 mg/ml, 0.05 mg/ml to 15 mg/ml, 0.05 mg/ml to 10 mg/ml, 0.05 mg/ml to 8 mg/ml, or 0.05 mg/ml to 3 mg/ml. In an embodiment, the liquid formulation may include a surfactant in an amount of 0.05 mg/ml to 50 mg/ml and amino acids in an amount of 0.05 mg/ml to 8 mg/ml. In an embodiment, the liquid formulation may include polysorbates or poloxamers in an amount of 0.05 mg/ml to 50 mg/ml and amino acids in an amount of 0.05 mg/ml to 8 mg/ml.

In an embodiment, the liquid formulation may include sodium chloride in an amount ranging from 0.1 mg/ml to 50 mg/ml, 0.5 mg/ml to 50 mg/ml, 1 mg/ml to 50 mg/ml, 1 mg/ml to 40 mg/ml, 0.1 mg/ml to 30 mg/ml, 0.1 mg/ml to 20 mg/ml, 0.1 mg/ml to 15 mg/ml, 0.1 mg/ml to 10 mg/ml, 0.5 mg/ml to 30 mg/ml, 0.5 mg/ml to 20 mg/ml, 3 mg/ml to 15 mg/ml, 3 mg/ml to 20 mg/ml, or 5 mg/ml to 15 mg/ml.

The botulinum toxin formulation disclosed herein may be formulated in any form, such as a solid or liquid formulation, for example, as a lyophilized powder, a liquid, or a pre-filled syringe formulation.

In an embodiment, the botulinum toxin formulation is a lyophilized preparation or a liquid preparation.

In an embodiment, the botulinum toxin is selected from the group consisting of botulinum toxin serotypes A, B, C, D, E, F, and G. For example, botulinum toxin is botulinum toxin serotype A.

In an embodiment, the botulinum toxin formulation is intended for improving symptoms such as wrinkles, a square jaw, a pointed jaw, wounds, skin softening, scars, acne, pores, elasticity, or keloids, or for treating conditions such as facial spasms, eyelid spasms, torticollis, blepharospasm, cervical dystonia, oropharynx dystonia, spasmodic dysphonia, migraines, pruritis ani, or hyperhidrosis.

The following describes methods related to the formulation in an embodiment. Unless otherwise stated, the content described for the formulation above is equally applicable to the methods related to the formulation described below.

According to an embodiment, a method of manufacturing the botulinum toxin formulation is provided, which includes mixing a botulinum toxin with a pharmaceutically acceptable excipient to obtain a botulinum toxin formulation,

wherein the pharmaceutically acceptable excipient does not include albumin,
the botulinum toxin is used in an amount corresponding to a desired potency, and

and the amount corresponding to the desired potency includes an overage of less than 50%.

In an embodiment, the difference in the botulinum toxin overage (%) between a botulinum toxin formulation including animal proteins and the formulation may be 40% or greater.

In an embodiment, the pharmaceutically acceptable excipient may include a surfactant and an amino acid.

In an embodiment, the mixing a excipient may include mixing a surfactant in an amount of 0.05 mg/ml to 50 mg/ml and mixing an amino acid in an amount of 0.05 mg/ml to 8 mg/ml.

Alternatively, a method of reducing a potency loss of botulinum toxin during the manufacturing process is provided, which includes obtaining the botulinum toxin formulation.

According to another aspect, a method of improving or treating a disease is provided, which includes administering an effective amount of the botulinum toxin formulation to a subject in need thereof.

Alternatively, the method relates to reducing the immunogenicity of the botulinum toxin formulation, which includes administering an effective amount of the botulinum toxin formulation to a subject in need thereof.

In another aspect, there is provided a use of an effective amount of the botulinum toxin formulation in the manufacture of a medicament for therapeutic purposes.

In an embodiment, the botulinum toxin may be administered in a single or multiple treatment sessions. The dosage may be administered as a single injection or divided doses at the injection site. In multiple treatment sessions, the botulinum toxin may be administered at intervals of 6 months, 4 months, or 3 months or less. In multiple treatment sessions, the dosing interval includes a primary treatment and a secondary treatment, and the dosage of the secondary treatment may be less than, greater than, or equal to the dosage of the primary treatment.

The dosage, administration time, administration method, administration period or interval of botulinum toxin may be appropriately selected and used by a person skilled in the art based on the patient's weight, age, gender, health condition, and severity of disease.

The subject to which the botulinum toxin or a composition including it is administered may include, without limitation, humans or animals, such as humans, pigs, dogs, cats, cows, horses, and mice.

The present disclosure is described in more detail below by way of examples, which are intended only to illustrate the present disclosure and are not intended to limit its scope in any way.

### Example: Manufacture of lyophilized and liquid formulations of botulinum toxin with minimal potency loss during the manufacturing process

Potency assay, mouse $LD_{50}$

The potency assay was conducted as follows: For the lyophilized formulation, 2.8 mL of saline solution was added to each of the two sample vials to dissolve them (total 5.6 mL). Here, 0.6 mL of saline solution was added to make an initial dilution of 5.6 mL. The dilution process was conducted as follows: From the initial dilution solution, 4.4 mL was taken and added to 1.45 mL of saline to prepare Test Solution 1. Then, 4.4 mL of Test Solution 1 was taken and added to 1.45 mL of saline to prepare Test Solution 2. This process was repeated eight more times, with each solution being used as needed. Test Solutions 3 to 6 were administered intraperitoneally into 10 mice (CD1, female) weighing 17 to 22 g at a dose of 0.1 mL per mouse. Mortality was measured after 3 days and statistically analyzed using the Combistats method to calculate potency.

Control formulation: Lyophilized formulation of botulinum toxin A complex and sodium chloride (NaCl, 0.9%) and human serum albumin (HSA, 0.5%)

A pharmaceutical composition including botulinum toxin A complex (100 units (1000 units/mL), with 191 units of toxin input (91% overage) to manufacture a 100-unit toxin formulation) and excipient of sodium chloride (9.0 mg/mL) and human serum albumin (5.0 mg/mL) which is an animal-derived protein was prepared. The composition was manufactured by commercial manufacturing procedures, filled into 0.1 mL (100 units)/vial, and lyophilized to produce a powder form. Afterwards, it was reconstituted in saline solution and the potency was measured using the potency assay (mouse $LD_{50}$). The potency recovery rate (%) was calculated through the potency measured after lyophilization to the initial input potency, and based on this, the overage required during manufacturing was determined using the formula: Overage (%) = ((100 / Potency recovery rate) - 1) $\times$ 100.

Formulation 1

A pharmaceutical composition including 100 units of botulinum toxin A complex (1000 units/mL) and excipients of amino acid (0.05 to 8.0 mg/mL), surfactant (0.05 to 50 mg/mL), and sodium chloride (9.0 mg/mL) was prepared and formulated using commercial manufacturing procedures, and then filled into 0.1 mL/vial to prepare a liquid form. The amino acid used exemplarily in Formulation 1 is methionine, and the surfactant used exemplarily is polysorbate 20. Afterwards, the potency was measured using the potency assay (mouse $LD_{50}$). The potency recovery rate (%) was calculated by comparing the potency measured after liquid filling to the initial input potency. Based on this recovery rate, the required overage during manufacturing was determined using the formula:

$$Overage\ (\%) = ((100\ /\ Potency\ recovery\ rate) - 1) \times 100.$$

## Formulation 2

A pharmaceutical composition including 100 units of botulinum toxin A complex (1000 units/mL) and excipients of amino acid (0.05 to 8.0 mg/mL), surfactant (0.05 to 50 mg/mL), and sodium chloride (9.0 mg/mL) was prepared and formulated using commercial manufacturing procedures, and then filled into 0.1 mL/vial to prepare a liquid form. The amino acid used exemplarily in Formulation 1 is methionine, and the surfactant used exemplarily is polysorbate 80. Afterwards, the potency was measured using the potency assay (mouse $LD_{50}$). The potency recovery rate (%) was calculated by comparing the potency measured after liquid filling to the initial input potency. Based on this recovery rate, the required overage during manufacturing was determined using the formula:

$$Overage\ (\%) = ((100\ /\ Potency\ recovery\ rate) - 1) \times 100.$$

## Formulation 3

A pharmaceutical composition including 100 units of botulinum toxin A complex (1000 units/mL) and excipients of amino acid (0.05 to 8.0 mg/mL), surfactant (0.01 to 50 mg/mL), and sodium chloride (9.0 mg/mL) was prepared and prepared in liquid form by commercial manufacturing procedures and filling at 0.1 mL/vial. The amino acid used exemplarily in Formulation 1 is methionine, and the surfactant used exemplarily is poloxamer 188. Afterwards, the potency was measured using the potency assay (mouse LD50). The potency recovery rate (%) was calculated by comparing the potency measured after liquid filling to the initial input potency. Based on this recovery rate, the required overage during manufacturing was determined using the formula: Overage (%) = ((100 / Potency recovery rate) - 1) × 100.

Results

The results are shown in Table 1.

[Table 1]

| Formulation | Excipients (mg/mL) | | | | Overage Calculation | | | |
|---|---|---|---|---|---|---|---|---|
| | Huma n serum albumi n | Amino acid | Surfacta nt | Sodium chloride | Initial potenc y | Liquid formula tion materia l | Pote ncy reco very rate (%)* | Over age (%)** |
| Control formulation (Lyophilized) | 5 | N/A | N/A | 9 | 191 | 100 | 52.4 | 91.0 |
| | N/A | 0.05 | 0.05 | 9 | 100 | 87 | 87 | 14.9 |
| Formulation 1 (polysorbate 20) | N/A | 3 | 0.05 | 9 | 100 | 91.0 | 91.0 | 9.9 |
| | N/A | 0.05 | 50 | 9 | 100 | 124.4 | 124. 4 | 0.0 |
| | N/A | 3 | 50 | 9 | 100 | 109.5 | 109. 5 | 0.0 |
| | N/A | 8 | 50 | 9 | 100 | 82.7 | 82.7 | 20.9 |
| Formulation 2 Polysorbate 80) | N/A | 0.05 | 0.05 | 9 | 100 | 90 | 90 | 11.1 |
| | N/A | 0.05 | 50 | 9 | 100 | 101.8 | 101. 8 | 0.0 |
| | N/A | 3 | 50 | 9 | 100 | 101.0 | 101. 0 | 0.0 |
| | N/A | 8 | 50 | 9 | 100 | 85.2 | 85.2 | 17.4 |
| Formulation 3 Poloxamer 188) | N/A | 0.05 | 0.05 | 9 | 100 | 87.1 | 87.1 | 14.8 |
| | N/A | 0.05 | 50 | 9 | 100 | 112.0 | 112. 0 | 0.0 |
| | N/A | 3 | 50 | 9 | 100 | 94.3 | 94.3 | 6.0 |
| | N/A | 8 | 50 | 9 | 100 | 100.5 | 100. 5 | 0.0 |
| *: | Potency Recovery Rate = (semi-finished product potency/initial potency) X 100 | | | | | | | |
| **: | Overage = ((100 / Potency recovery rate)-1) X 100 | | | | | | | |

As shown in Table 1, the control formulation (a lyophilized formulation including botulinum toxin A complex, sodium chloride (NaCl, 0.9 %), and human serum albumin (HSA, 0.5%)) required a 91 % overage to compensate for potency loss during the manufacturing process. On the other hand, for Formulation 1 (liquid formulation including botulinum toxin A complex, methionine (0.05 mg/mL to 8.0 mg/mL), polysorbate 20 (0.05 mg/mL to 50 mg/mL), and sodium chloride (9.0 mg/mL)), it was confirmed that an overage of 0 % to 20.9 % was required depending on the concentration combination, demonstrating at least a 77% improvement in overage compared to the control formulation. For Formulation 2 (liquid formulation including botulinum toxin A complex, methionine (0.05 mg/mL to 8.0 mg/mL), polysorbate 80 (0.05 mg/mL to 50 mg/mL), and sodium chloride (9.0 mg/mL)), it was confirmed that an excess dosage of 0 to 17.4 % was required depending on the concentration combination, demonstrating an improvement of 81 % compared to the control formulation. For Formulation 3 (liquid formulation including botulinum toxin A complex, methionine (0.05 to 8.0 mg/mL), poloxamer 188 (0.05 to 50 mg/mL), and sodium chloride (9.0 mg/mL)), it was confirmed that an overage of 0 % to 14.8% was required depending on the concentration combination. Through this, it was confirmed that an 84 % improvement was achieved compared to the control group.

Thus, it was confirmed that liquid formulations including amino acids and nonionic surfactants demonstrated reduced inactivation of toxin proteins during the manufacturing process, leading to improved overage compared to the control formulation (which contained animal-derived proteins and NaCl).

Formulation 4

A pharmaceutical composition including 100 units of botulinum toxin A complex (1000 units/mL) and excipients of amino acid (0.05 to 8.0 mg/mL), surfactant (0.05 to 50 mg/mL), sodium chloride (9.0 mg/mL), and sucrose (30 mg/mL) was prepared. The composition was manufactured through commercial production procedures, filled into vials at a volume of 0.1 mL per vial, and lyophilized to produce a powder form. The amino acid used exemplarily in Formulation 4 is methionine, and the surfactant used exemplarily is polysorbate 20. Afterwards, the powder was reconstituted with saline, and potency was measured using the potency assay (mouse $LD_{50}$). The potency recovery rate (%) was calculated by comparing the potency measured after lyophilization to the initial input potency. Based on this recovery rate, the required overage during manufacturing was determined using the formula: Overage (%) = ((100 / Potency recovery rate) - 1) $\times$ 100.

Formulation 5

A lyophilized powder formulation was prepared using the same method as Formulation 4, except that the type of surfactant was changed to polysorbate 80. Afterwards, the powder was reconstituted with saline, and potency was measured using the potency assay (mouse $LD_{50}$). The potency recovery rate (%) was calculated by comparing the potency measured after lyophilization to the initial input potency. Based on this recovery rate, the required overage during manufacturing was determined using the formula

: Overage (%) = ((100 / Potency recovery rate) - 1) × 100.

Formulation 6

A lyophilized powder formulation was prepared in the same manner as Formulation 4, except that the type of surfactant used as an example was changed to poloxamer 188. Afterwards, the powder was reconstituted with saline, and potency was measured using the potency assay (mouse $LD_{50}$). The potency recovery rate (%) was calculated by comparing the potency measured after lyophilization to the initial input potency. Based on this recovery rate, the required overage during manufacturing was determined using the formula: Overage (%) = ((100 / Potency recovery rate) - 1) $\times$ 100.

Results

The results are shown in Table 2.

[Table 2]

| Formulation | Excipient | | | | | Overage Calculation | | | |
|---|---|---|---|---|---|---|---|---|---|
| | human serum albumin | Amino acid | Surfactant | Sodium chloride | Sucrose | Initial potency | Lyophilized material | Potency recovery rate (%)* | Overage (%)** |
| Control formulation | 5 | N/A | N/A | 9 | N/A | 191 | 100 | 52.4 | 91.0 |
| Formulation 4 (Poly-sorbate 20) | N/A | 0.05 | 0.05 | 9 | 30 | 100 | 79 | 79 | 26.6 |
| | N/A | 8 | 50 | 9 | 30 | 100 | 107 | 107 | 0.0 |
| Formulation 5 (Poly-sorbate 80) | N/A | 8 | 50 | 9 | 30 | 100 | 101 | 101 | 0.0 |
| Formulation 6 (Polox-amer 188) | N/A | 8 | 50 | 9 | 30 | 100 | 109.9 | 109.9 | 0.0 |

*: Potency recovery rate = (semi-finished product potency/initial potency) X 100

**: Overage = ((100 / Potency recovery rate)-1) X 100

EP 4 582 083 A1

As shown in Table 2, the control formulation (botulinum toxin A complex, sodium chloride (NaCl, 0.9 %), and human serum albumin (HSA, 0.5 %), lyophilized formulation) was found to require 91 % excess input to compensate for potency loss during the manufacturing process. In contrast, Formulation 4 (a lyophilized formulation including botulinum toxin A complex, methionine (0.05 mg/mL to 8.0 mg/mL), polysorbate 20 (0.05 mg/mL to 50 mg/mL), sodium chloride (9.0 mg/mL), and sucrose (30 mg/mL)) required an overage of 0 % to 26.6 %. For Formulation 5 and Formulation 6 (identical to the second composition of Formulation 4 except for the type of surfactant used), no overage was required for botulinum toxin. These results confirm that the lyophilized formulations provided here demonstrated reduced toxin protein inactivation during the manufacturing process, leading to improved overage reduction.

A botulinum toxin formulation free of animal proteins according to an embodiment, includes an amount of botulinum toxin corresponding to a desired potency, where the amount corresponding to the desired potency includes an overage of less than 50%. As a result, when administered to a subject, this formulation may suppress immune responses and reduce or inhibit the generation of neutralizing antibodies compared to formulations including animal-derived proteins. Therefore, the formulation in an embodiment reduces resistance expression within the body, allowing for consistent and repeated use without a loss of efficacy.

**Claims**

1.  A botulinum toxin formulation free of animal protein, comprising a botulinum toxin in an amount corresponding to a desired potency, wherein the amount corresponding to the desired potency comprises an overage of less than 50 %.

2.  The botulinum toxin formulation of claim 1, wherein the formulation comprises an overage of botulinum toxin in an amount less than the overage of botulinum toxin in a botulinum toxin formulation including animal protein.

3.  The botulinum toxin formulation of any one of claim 1 and claim 2, wherein the overage is less than 30%.

4.  The botulinum toxin formulation of any one of claim 1 to claim 3, being a lyophilized formulation or a liquid formulation.

5.  The botulinum toxin formulation of claim 4, wherein the lyophilized formulation comprises a botulinum toxin, a surfactant, an amino acid, and one or more components selected from the group consisting of sugars, sugar alcohols, and ionic compounds.

6.  The botulinum toxin formulation of claim 5, wherein the surfactant comprises at least one of polysorbate and poloxamer.

7.  The botulinum toxin formulation of claim 5, wherein the amino acid comprises methionine.

8.  The botulinum toxin formulation of claim 5, wherein the lyophilized formulation comprises 0.05 mg/ml to 50 mg/ml of a surfactant and 0.05 mg/ml to 8 mg/ml of an amino acid.

9.  The botulinum toxin formulation of claim 4, wherein the liquid formulation comprises a botulinum toxin, a surfactant, and an amino acid.

10. The botulinum toxin formulation of claim 9, wherein the surfactant comprises one or more of polysorbate and poloxamer.

11. The botulinum toxin formulation of claim 9, wherein the amino acid comprises methionine.

12. The botulinum toxin formulation of claim 9, wherein the liquid formulation comprises 0.05 mg/ml to 50 mg/ml of a surfactant and 0.05 mg/ml to 8 mg/ml of an amino acid.

13. The botulinum toxin formulation of any one of claim 1 to claim 12, wherein the botulinum toxin is selected from the group consisting of botulinum toxin serotypes A, B, C, D, E, F, and G.

14. The botulinum toxin formulation of any one of claim 1 to claim 13, being for improving wrinkles, a square jaw, a pointed jaw, wounds, skin softening, scars, acne, pores, elasticity, or keloids, or for treating facial spasms, eyelid spasms, torticollis, blepharospasm, cervical dystonia, oropharynx dystonia, spasmodic dysphonia, migraines, pruritis ani, or hyperhidrosis.

**15.** The botulinum toxin formulation of any one of claim 1 to claim 14, having reduced immunogenicity.

**16.** A method of manufacturing a botulinum toxin formulation, comprising:

mixing a botulinum toxin with a pharmaceutically acceptable excipient to obtain the botulinum toxin formulation,
wherein the pharmaceutically acceptable excipient is free of albumin,
wherein the botulinum toxin is used in an amount corresponding to a desired potency, and
wherein the amount corresponding to the desired potency comprises an overage of less than 50%.

**17.** The method of claim 16, wherein the formulation comprises an overage of botulinum toxin in an amount less than the overage of botulinum toxin in a botulinum toxin formulation including animal protein.

**18.** The method of any one of claim 16 and claim 17, wherein the pharmaceutically acceptable excipient includes a surfactant and an amino acid.

**19.** The method of any one of claim 16 to claim 18, wherein the mixing with an excipient comprises mixing 0.05 mg/ml to 50 mg/ml of a surfactant and mixing 0.05 mg/ml to 8 mg/ml of an amino acid.

**20.** A method of reducing a potency loss of a botulinum toxin during a manufacturing process, comprising obtaining the botulinum toxin formulation of any one of claim 1 to claim 15.

**21.** A method of reducing immunogenicity of a botulinum toxin formulation, comprising administering to a subject in need thereof an effective amount of the botulinum toxin formulation of any one of claim 1 to claim 15.

**22.** A method of improving symptoms of wrinkles, a square jaw, a pointed jaw, wounds, skin softening, scars, acne, pores, elasticity, or keloids, or treating facial spasms, eyelid spasms, torticollis, blepharospasm, cervical dystonia, oropharynx dystonia, spasmodic dysphonia, migraines, pruritis ani, or hyperhidrosis, comprising administering to a subject in need thereof an effective amount of the botulinum toxin formulation of any one of claim 1 to claim 13.

**23.** Use of an effective amount of the botulinum toxin formulation of any one of claim 1 to claim 13 in the manufacture of a medicament for improving symptoms of wrinkles, a square jaw, a pointed jaw, wounds, skin softening, scars, acne, pores, elasticity, or keloids, or for treating facial spasms, eyelid spasms, torticollis, blepharospasm, cervical dystonia, oropharynx dystonia, spasmodic dysphonia, migraines, pruritis ani, or hyperhidrosis.

## INTERNATIONAL SEARCH REPORT

| International application No. |
| --- |
| **PCT/KR2023/013194** |

### A. CLASSIFICATION OF SUBJECT MATTER

**A61K 9/19**(2006.01)i; **A61K 47/26**(2006.01)i; **A61K 47/20**(2006.01)i; **A61K 9/08**(2006.01)i; **A61K 38/48**(2006.01)i; **A61K 8/66**(2006.01)i; **A61Q 19/00**(2006.01)i

According to International Patent Classification (IPC) or to both national classification and IPC

### B. FIELDS SEARCHED

Minimum documentation searched (classification system followed by classification symbols)

A61K 9/19(2006.01); A61K 35/74(2006.01); A61K 38/00(2006.01); A61K 38/16(2006.01); A61K 38/38(2006.01); A61K 38/48(2006.01); A61K 47/18(2006.01); A61K 47/22(2006.01); A61K 9/00(2006.01); A61K 9/08(2006.01); C07K 14/33(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 보톡스; 보툴리눔 독소(botox; botulinum toxin), 역가(potency), 초과투입 (overage), 알부민(albumin), 메티오닌(methionine), 계면활성제(surfactant)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2022-0071163 A (MEDYTOX INC.) 31 May 2022 (2022-05-31)<br>See claims 1-11 and paragraph [0077]. | 1-3,16-18 |
| X | KR 10-1357999 B1 (HAM, Jong Wook) 03 February 2014 (2014-02-03)<br>See claims 1-3. | 1-3,16-18 |
| DX | WO 2015-089452 A1 (ALLERGAN, INC.) 18 June 2015 (2015-06-18)<br>See paragraphs [0024]-[0058]. | 1-3,16-18 |
| A | KR 10-2022-0003061 A (OBI PHARMA, INC.) 07 January 2022 (2022-01-07)<br>See claims 1-21. | 1-3,16-18 |
| A | KR 10-2021-0130711 A (MERZ PHARMA GMBH & CO. KGAA) 01 November 2021 (2021-11-01)<br>See claims 1-20. | 1-3,16-18 |

☑ Further documents are listed in the continuation of Box C.　　☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **21 December 2023** | **22 December 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/013194** |

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2005-0094817 A (BOTULINUM TOXIN RESEARCH ASSOCIATES, INC.) 28 September 2005 (2005-09-28)<br>   See claims 1-60. | 1-3,16-18 |

Form PCT/ISA/210 (second sheet) (July 2022)

**INTERNATIONAL SEARCH REPORT**

| International application No. |
|---|
| **PCT/KR2023/013194** |

---

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **21,22**
   because they relate to subject matter not required to be searched by this Authority, namely:

   Claims 21 and 22 pertain to a method for treatment of the human body [PCT Article 17(2)(a)(i) and PCT Rule 39.1(iv)].

2. ☑ Claims Nos.: **5-12**
   because they relate to parts of the international application that do not comply with the prescribed requirements to such an extent that no meaningful international search can be carried out, specifically:

   Claims 5-12 refer to claims which do not comply with PCT Rule 6.4(a) and are not established (PCT Article 6).

3. ☑ Claims Nos.: **4,13-15,19-23**
   because they are dependent claims and are not drafted in accordance with the second and third sentences of Rule 6.4(a).

---

Form PCT/ISA/210 (continuation of first sheet) (July 2022)

## INTERNATIONAL SEARCH REPORT
### Information on patent family members

International application No.

**PCT/KR2023/013194**

| Patent document cited in search report | | | Publication date (day/month/year) | Patent family member(s) | | | Publication date (day/month/year) |
|---|---|---|---|---|---|---|---|
| KR | 10-2022-0071163 | A | 31 May 2022 | BR | 112022020327 | A2 | 13 December 2022 |
| | | | | CN | 115397399 | A | 25 November 2022 |
| | | | | EP | 4122448 | A1 | 25 January 2023 |
| | | | | KR | 10-2021-0125354 | A | 18 October 2021 |
| | | | | KR | 10-2021-0125358 | A | 18 October 2021 |
| | | | | KR | 10-2021-0125359 | A | 18 October 2021 |
| | | | | KR | 10-2404217 | B1 | 02 June 2022 |
| | | | | KR | 10-2486484 | B1 | 10 January 2023 |
| | | | | WO | 2021-206360 | A1 | 14 October 2021 |
| KR | 10-1357999 | B1 | 03 February 2014 | KR | 10-2013-0106557 | A | 30 September 2013 |
| WO | 2015-089452 | A1 | 18 June 2015 | AU | 2014-361859 | A1 | 18 June 2015 |
| | | | | AU | 2020-203349 | A1 | 11 June 2020 |
| | | | | AU | 2022-231718 | A1 | 06 October 2022 |
| | | | | CA | 2933363 | A1 | 18 June 2015 |
| | | | | CN | 106163545 | A | 23 November 2016 |
| | | | | CN | 116570567 | A | 11 August 2023 |
| | | | | EP | 3079714 | A1 | 19 October 2016 |
| | | | | EP | 3079714 | B1 | 25 November 2020 |
| | | | | EP | 3845241 | A1 | 07 July 2021 |
| | | | | EP | 3845241 | A8 | 18 August 2021 |
| | | | | ES | 2861509 | T3 | 06 October 2021 |
| | | | | JP | 2016-540785 | A | 28 December 2016 |
| | | | | JP | 2020-045360 | A | 26 March 2020 |
| | | | | JP | 2022-161932 | A | 21 October 2022 |
| | | | | JP | 6895751 | B2 | 30 June 2021 |
| | | | | JP | 7121458 | B2 | 18 August 2022 |
| | | | | KR | 10-2016-0113597 | A | 30 September 2016 |
| | | | | KR | 10-2022-0071292 | A | 31 May 2022 |
| | | | | KR | 10-2404218 | B1 | 02 June 2022 |
| | | | | US | 10143728 | B2 | 04 December 2018 |
| | | | | US | 2020-397875 | A1 | 24 December 2020 |
| | | | | US | 2023-149520 | A1 | 18 May 2023 |
| | | | | US | 9480731 | B2 | 01 November 2016 |
| KR | 10-2022-0003061 | A | 07 January 2022 | AU | 2020-440362 | A1 | 04 November 2021 |
| | | | | AU | 2020-440362 | B2 | 16 February 2023 |
| | | | | BR | 112021024058 | A2 | 11 October 2022 |
| | | | | CN | 114222754 | A | 22 March 2022 |
| | | | | CN | 116041452 | A | 02 May 2023 |
| | | | | EP | 3932938 | A1 | 05 January 2022 |
| | | | | EP | 3932938 | A4 | 30 November 2022 |
| | | | | JP | 2022-548189 | A | 17 November 2022 |
| | | | | JP | 7328714 | B2 | 17 August 2023 |
| | | | | MX | 2021014486 | A | 04 January 2022 |
| | | | | NZ | 780855 | A | 30 June 2023 |
| | | | | SG | 11202111282 | A | 29 November 2021 |
| | | | | US | 2022-257730 | A1 | 18 August 2022 |
| | | | | WO | 2021-195968 | A1 | 07 October 2021 |
| KR | 10-2021-0130711 | A | 01 November 2021 | AU | 2020-226945 | A1 | 27 August 2020 |
| | | | | AU | 2020-226945 | A1 | 22 July 2021 |
| | | | | CA | 3130411 | A1 | 27 August 2020 |

Form PCT/ISA/210 (patent family annex) (July 2022)

## INTERNATIONAL SEARCH REPORT
### Information on patent family members

International application No.

**PCT/KR2023/013194**

| Patent document cited in search report | | | Publication date (day/month/year) | Patent family member(s) | | | Publication date (day/month/year) |
|---|---|---|---|---|---|---|---|
| | | | | CN | 113573727 | A | 29 October 2021 |
| | | | | EP | 3927355 | A1 | 29 December 2021 |
| | | | | IL | 285634 | A | 30 September 2021 |
| | | | | JP | 2022-521237 | A | 06 April 2022 |
| | | | | MX | 2021009922 | A | 14 September 2021 |
| | | | | SG | 11202107565 | A | 30 August 2021 |
| | | | | US | 2022-0143157 | A1 | 12 May 2022 |
| | | | | WO | 2020-169578 | A1 | 27 August 2020 |
| KR | 10-2005-0094817 | A | 28 September 2005 | AT | 426408 | T | 15 April 2009 |
| | | | | AU | 2003-300666 | A1 | 29 July 2004 |
| | | | | AU | 2010-202062 | A1 | 10 June 2010 |
| | | | | BR | 0316871 | A | 18 October 2005 |
| | | | | CA | 2510058 | A1 | 22 July 2004 |
| | | | | CA | 2510058 | C | 16 March 2010 |
| | | | | CN | 1729011 | A | 01 February 2006 |
| | | | | CN | 1729011 | C | 01 February 2006 |
| | | | | CR | 9813 | A | 29 July 2008 |
| | | | | EP | 1594523 | A2 | 16 November 2005 |
| | | | | EP | 1678881 | B1 | 01 May 2013 |
| | | | | EP | 1678881 | B8 | 28 January 2015 |
| | | | | IL | 168745 | A0 | 01 December 2011 |
| | | | | IL | 168745 | B | 29 December 2011 |
| | | | | IL | 168745 | D0 | 01 December 2011 |
| | | | | JP | 2006-519761 | A | 31 August 2006 |
| | | | | MX | PA05006690 | A | 17 February 2006 |
| | | | | NO | 20053424 | A | 16 September 2005 |
| | | | | NO | 20053424 | D0 | 14 July 2005 |
| | | | | NO | 20053424 | L | 16 September 2005 |
| | | | | NZ | 540425 | A | 31 March 2009 |
| | | | | US | 2003-0231608 | A1 | 18 December 2003 |
| | | | | US | 2011-0172161 | A1 | 14 July 2011 |
| | | | | US | 2014-0133378 | A1 | 15 May 2014 |
| | | | | US | 2014-0315820 | A1 | 23 October 2014 |
| | | | | US | 6791962 | B2 | 14 September 2004 |
| | | | | US | 8446933 | B2 | 21 May 2013 |
| | | | | US | 8787988 | B2 | 22 July 2014 |
| | | | | US | 9002415 | B2 | 07 April 2015 |
| | | | | US | RE043127 | E1 | 24 January 2012 |
| | | | | US | RE045212 | E1 | 28 October 2014 |
| | | | | US | RE43127 | E1 | 24 January 2012 |
| | | | | WO | 2004-060384 | A2 | 22 July 2004 |
| | | | | WO | 2005-067535 | A2 | 28 July 2005 |
| | | | | WO | 2005-067535 | A3 | 08 March 2007 |
| | | | | WO | 2006-116302 | A2 | 02 November 2006 |
| | | | | ZA | 200504982 | A | 30 May 2007 |
| | | | | ZA | 200504982 | B | 30 May 2007 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015089452 A

- WO 2007016018 A